# NEUE EUROPÄISCHE PATENTSCHRIFT

(11) **EP 1 392 337 B2**
(45) Veröffentlichungstag und Bekanntmachung des Hinweises auf die Entscheidung über den Einspruch: **22.07.2009**
(45) Hinweis auf die Patenterteilung: 30.11.2005
(21) Anmeldenummer: 02747268.7
(22) Anmeldetag: 10.04.2002
(51) Int. Cl.: A61K 36/18, A61K 9/24, A61K 33/10, A61P 19/10

(54) **PHARMAZEUTISCHE FORMULIERUNG BESTEHEND AUS EINEM PFLANZENTROCKENEXTRAKT MIT EINER CALCIUMUMMANTELUNG**
PHARMACEUTICAL FORMULATION CONSISTING OF A PLANT DRY EXTRACT WITH A CALCIUM COATING
FORMULATION PHARMACEUTIQUE A BASE D'UN EXTRAIT SEC DE PLANTE, MUNI D'UN ENROBAGE CALCIUM

(30) Priorität: 08.06.2001 DE 10127897
(43) Veröffentlichungstag der Anmeldung: 03.03.2004
(73) Patentinhaber: Bionorica AG, 92318 Neumarkt (DE)
(72) Erfinder: POPP, Michael, A., 92318 Neumarkt (DE)
(74) Vertreter: Simandi, Claus
(86) Internationale Anmeldenummer: PCT/EP2002/004002
(87) Internationale Veröffentlichungsnummer: WO 2002/100422

(56) Entgegenhaltungen:
- EP-B1- 1 392 337
- WO-A-00/06127
- WO-A-00/30605
- KR-B- 267 576
- US-A- 5 093 130
- US-A- 5 569 459
- 'Rote Liste', 1999, EDITIO CANTOR VERLAG, AULENDORF Artikel '49037 "Kytta-Sedativum"'
- 'Rote Liste', 1999, EDITIO CANTOR VERLAG, AULENDORF Artikel '60325 "Uzara"'
- PRINCE RL, KERR DA MED J AUST. Bd. 173, Nr. SUPPL, 06 November 2000, Seiten 106 - 107
- R. VOIGT: 'Lehrbuch der pharmazeutischen Technologie', 1973, VEB VERLAG VOLK UND GESUNDHEIT, BERLIN
- 'Hagers Handbuch der Pharmazeutischen Praxis', Bd. 7. BAND, teil A 1971, SPRINGER-VERLAG, BERLIN-HEIDELBERG-NEW YORK Seiten 674 - 689,724
- 'Deutsches Arzneibuch', Bd. 7. AUSG., 1968, DEUTSCHER APOTHEKER-VERLAG, STUTTGART Seiten 924 - 925
- WOLFGANG PFEIFER: 'Der Pharmakant', 1989, WISSENSCHAFTLICHE VERLAGSGESELLSCHAFT MBH, STUTTGART Seiten 159 - 161
- BAUER, LEHMANN, OSTERWALD, ROTHGANG: 'Überzogene Arzneiformen', 1989, WISSENSCHAFTLICHE VERLAGSGESELLSCHAFT MBH, STUTTGART Seiten 58 - 62

## Beschreibung

Die vorliegende Erfindung betrifft eine pharmazeutische Formulierung gemäß dem Oberbegriff des Anspruchs 1, eine Verwendung derselben gemäß Anspruch 11 sowie ein Verfahren zu ihrer Herstellung gemäß Anspruch 13.

Das natürlich in den Ovarien gebildete 17β-Estradiol [auch mit E₂ bezeichnet] hat im menschlichen und tierischen Organismus eine allgemein proliferationsfördemde Wirkung. Neben der Steuerung des Zyklus' der Frau besitzt es u.a. einen homöostatischen Einfluß auf den Stoffwechsel des Knochens und verhindert am Endothel der Gefäße die Entstehung atherotischer Plaques.

In der Menopause kommt es zu einem Absinken der Estradiotspiegel in Folge des Erlöschens der Ovarialfunktion. In Abwesenheit genügend hoher Estradiolspiegel im Blut überwiegt im Knochengewebe die Aktivität der Osteoklasten und damit der Abbau der Knochenmasse - der sogenannten Osteoporose - die mit erhöhter Bruchgefahr des Skeletts einhergeht.

In jüngerer Zeit hat sich herausgestellt, daß dieses Sexualhormonmangel-bedingte Krankheitsbild der Osteoporose nicht auf Frauen beschränkt ist, sondem daß es ab einem gewissen fortgeschritten Alter, insbesondere im Zusammenhang mit Erkrankungen der Prostata, auch beim Mann zu Sexualhormonmangel-bedingter Osteoporose kommt.

Die klassische Prophylaxe und Therapie bei der Frau besteht in der Substitution des fehlenden natürlichen Östrogens durch Gabe von natürlichen und synthetischen Östrogenen. Insbesondere zur Prophylaxe werden bei Mann und Frau Calciumgaben von ca. 1 Gramm Calcium pro Tag appliziert.

Die Gabe von Östrogenen wie zB. 17β-Estradiol oder dessen chemischen Abkömmlingen geht jedoch mit den bekannten gravierenden Nebenwirkungen wie uterotrope Wirkung, erhöhtes Thromboembolierisiko, Gewichtszunahme usw. einher.

Daher wurden im Stand der Technik viele Versuche unternommen, Mittel zu finden, welche in der Lage sind, eine estrogenarüge Wirkung hervorzurufen, ohne oder jedoch mit stark verminderten unerwünschten Nebenwirkungen. Hierbei kamen häufig Pflanzenextrakte zum Einsatz, welche die gewünschte Osteoporoseprophylaxewirkung nicht jedoch die unerwünschten uterotropen Wirkungen zeigten.

So beschreibt beispielsweise die WO 99/47149 (=EP 1064009A1) der Anmelderin der vorliegenden Anmeldung, daß Extrakte aus Irisgewächsen, insbesondere aus Cimicifuga racemosa und Belamcanda chinensis und des darin enthaltenen Isoflavonoids Tectorigenin keine östrogene Wirkung am Uterus, wohl aber in der hypothalamo-hypophysären Achse, im Herz-Kreislauf-System und im Knochen ausüben, so daß diese Pflanzenextrakte zur Osteoporoseprophylaxe und -therapie eingesetzt werden können.

Desweiteren sind im Stand der Technik Calciumpräparate zur Osteoporoseprophylaxe bekannt. Als pharmazeutische Formulierung hierfür kommt neben den Injektionslösungen zur oralen Anwendung vorzugsweise eine Brausetablette in Betracht. Derartige Brausetabletten können beispielsweise folgende Zusammensetzung aufweisen:
Tabletten mit 500 mg Ca2+: Calciumcarbonat 1250 mg, Citronensäure 2050 mg, weitere Bestandteile: Lactose 1H2O, Macrogol 6000, Povidon, Natriumcyclamat, Saccharin-Natrium 2H2O, Dimeticon 350, hochdisperses Siliciumdioxid, Macrogolstearat 400, Sorbinsäure, Aromastoffe.
Tabletten mit 1000 mg Ca2+: Calciumlactogluconat 4954 mg, Calciumcarbonat 900 mg, weitere Bestandteile: Citronensäure, Natriumcyclamat, Saccharin-Natrium, Mannitol, Macrogol 4000, Natriumhydrogencarbonat, Aromastoffe.

Um Patienten keine Mehrfacheinnahmen zuzumuten und aus Kostengesichtspunkten wäre eine Kombination von Calciumpräparaten, die einen hohen Ca²⁺-Gehalt aufweisen, mit Pflanzentrockenextrakten mit anti-Osteoporosewirkung wünschenswert.

Die einfache Beimischung zu einer Calcium-Brausetablettenmasse scheitert jedoch einerseits an der großen Empfindlichkeit der Trockenextrakte gegenüber Säuren im wäßrigen Medium, andererseits an der mäßigen Wasserlöslichkeit vieler Trockenextrakte im sauren Medium. Ferner sind die feuchtigkeitsempfindlichen Brausemassen durch die starke Hygroskopie der Trockenextrakte gefährdet.

Eine Verkapselung in üblichen Gelatinekapseln kommt ebenfalls nicht in Betracht, da die Kapseln in der Regel nicht wasserdampfdicht sind und somit den Pflanzentrockenextrakt zersetzen und/oder verklumpen würden, so daß die pharmazeutische Qualität wegen undefinierter

Bioverfügbarkeit, Freisetzung und/oder Wirksamkeit nicht mehr gewährleistet wäre. Ferner ist die Stabilität derartiger Gelatinekapseln nicht gegeben, da in der Literatur berichtet wurde, daß beispielsweise Quervemetzungen mit der Gelatine auftreten, wenn Pflanzenextrakte in Gelatine verkapselt werden und sich durch Wasseraufnahme des Trockenextraktes gravierende Veränderungen der Inhaltsstoffe ergeben.

Optimal wäre daher beispielsweise eine Drageeformulierung, welche aus einer Mischung aus Calcium und Pflanzentrockenextrakt bestünde. Wie oben angegeben, würden sich bei signifikantem Calciumgehalt ab 500 mg Ca²⁺ zusammen mit den für die Drageeherstellung relativ großen Hilfsstoffmengen jedoch Drageegrößen und -gewichte ergeben, die für Patienten nicht mehr einnehmbar wären.

Als feste Formulierung sind Kombinationspräparate von Calcium mit Vitamin D3 in Form von Kautabletten , zB. als Ossofortin® forte Kautabletten der Firma Strathmann AG + Co., Sellhapsweg 1, 22459 Hamburg, bekannt.

Derartige Kautabletten zur unterstützenden Behandlung der Osteoporose weisen folgende Zusammensetzung auf:
Calciumcarbonat 1500,3 mg (entspr. 600 mg Ca++). Colecalciferol-Trockenkonzentrat (100 000 I.E./g) 400 I.E., weitere Bestandteile: Xylitol, Maisstärke, Saccharin-Natrium, Aroma, Magnesiumstearat.

Die Druckschrift KR267576, die inhaltlich der Druckschrift US2001036468 entspricht, handelt von einer Multivitaminkautablette, die aus einem Kern und einer äußeren Multilaminatschicht besteht. Der Kern kann 30-50 mg Fruchtsaftpulver enthalten und von einer CaPO₄- und Vitamin D3-haltigen Hülle umgeben sein. Der Kern liegt hierbei in Form von losem Fruchtsaftpulver vor.

Die Druckschrift WO0006127 beschreibt eine Zubereitung, bestehend aus einem pflanzlichem Flüssigextrakt, der von einer mineralhaltigen Hülle (z.B. Calciumchlorid, Calciumgluconat) umgeben wird. Als Pflanzenextrakte werden Flüssigextrakte von beispielsweise Echinacea genannt.

Die Druckschrift US5093130 beschreibt Hydrogelkapseln, die pflanzlichen Bestandteile enthalten und mit einem Überzug aus Calciumstearat versehen sind. Verwendete pflanzliche Gewebe sind beispielsweise Tomatensamen.

Die Druckschriften WO0030605 und US5569459 beschreiben pharmazeutische Zubereitungen, wie beispielsweise Tabletten, die einen pflanzlichen Trockenextrakt enthalten. Calciumsalze werden mit dem Trockenextrakt vermischt und gemeinsam zu Tabletten verpreßt.

Eine Kombination von Calciumpräparaten mit Pflanzenextrakten, welche zur eingangs geschilderten Osteoporoseprophylaxe und/oder - therapie geeignet sein soll, gibt es aus den geschilderten Gründen bislang im Stand der Technik nicht.

Daher ist es Aufgabe der vorliegenden Erfindung, eine pharmazeutische Formulierung zur Verfügung zu stellen, welche sowohl die Vorteile einer Calciumsubstitution und bei Bedarf Vitamin D₃ - Substitution als auch die Vorteile eines Pflanzentrockenextraktes zur Prophylaxe und/oder Behandlung der Osteoporose vereint.

Diese Aufgabe wird durch die kennzeichnenden Merkmale des Anspruchs 1 gelöst. Verwendungstechnisch wird die Aufgabe durch die Merkmale des Anspruchs 11 und verfahrenstechnisch durch die kennzeichnenden Merkmale des Anspruchs 13 gelöst.

Die Erfindung betrifft insbesondere eine pharmazeutische Formulierung gemäß Anspruch 1.

Mit der erfindungsgemäßen pharmazeutische Formulierung ist es erstmals möglich, mengenmäßig sinnvolle Kombinationen zwischen Osteoporose-wirksamen pflanzlichen Trockenextrakten, wie beispielsweise Cimicifuga racemosa, Belamcanda chinensis, Vitex agnus castus oder Urtica dioica radix sowie deren Mischungen, mit Calcium zur Verfügung zu stellen. Hierbei ist es besonders vorteilhaft, daß der Mantel - nicht wie im Stand der Technik üblich - aus Hilfsstoffen besteht, sondern im wesentlichen ausschließlich Wirkstoff, nämlich Calcium, enthält.

Zur Herstellung der Pflanzentrockenextrakte wird vollinhaltlich auf die PCT-Anmeldung der vorliegenden Anmelderin WO 99/47149 verwiesen. Neben den dort angesprochenen Lösungsmitteln ist es jedoch auch noch möglich mit Mischungen aus organisch-wäßrigen Lösungsmitteln, ausschließlich wäßrig oder auch mittels überkritischem CO₂ (z.B. im Falle von Serenoa repens) die in Rede stehenden Pflanzen zu extrahieren.

Mit der vorliegenden pharmazeutischen Formulierung wird erreicht, daß die in der Regel sehr hygroskopischen Pflanzenextrakte, welche normalerweise durch Zugabe von wasserbindenden Hilfsstoffen z.B. zu Dragees oder Filmtabletten formuliert werden, mit kleiner Masse und kleinem Volumen, eingebettet in einen Mantel aus einem inerten, vor Feuchtigkeit schützenden Wirkstoff in Form eines Calciumsalzes zur Verfügung gestellt werden können.

Hierdurch wird ein extrem günstiges Verhältnis von Pflanzentrockenextrakt/Calcium zu Hilfsstoffen erzielt, so daß teilweise sogar gänzlich auf Hilfsstoffe, mit Ausnahme geringer Mengen Schmierstoffe, verzichtet werden kann.

Hierdurch ist es erstmals möglich, ein Kombinationspräparat aus einem Pflanzentrockenextrakt, Calciumsalzen und bei Bedarf auch Vitamin D₃ zur Verfügung zu stellen, welches einen Calciumgehalt aufweist, mit der die zur Prophylaxe und/oder Therapie erforderlichen etwa 1000 mg Ca²⁺-lonen p.d. ohne Gabe zusätzlicher Calciumpräparate erreicht werden können.

Eine bevorzugte Ausführungsform der vorliegenden Erfindung ist dadurch gekennzeichnet daß sie im calciumhaltigen Mantel und/oder im inneren Pflanzentrockenextrakt-Preßling zusätzlich Cholecalciferole, insbesondere Colecalciferol (Vitamin D3) enthält.

Darüber hinaus kann die pharmazeutische Formulierung zusätzlich wenigstens ein Fluoridsalz, insbesondere Natriumfluorid enthalten, wobei es bevorzugt ist, daß .das Fluoridsalz im inneren Preßling vorliegt.

Bevorzugt ist die pharmazeutische Formulierung dadurch gekennzeichnet, daß der Pflanzentrockenextrakt ausgewählt ist aus der Gruppe, bestehend aus Extrakten von: Vitex agnus castus (Mönchspfeffer); Belamcanda chinensis (Leopardenlilie); Cimicifuga racemosa (Traubensilberkerze); Trifolium pratense L. (Rotklee); Oenothera biennis hom. (Nachtkerze); Glycine soja (Sojabohne); Serenoa repens (Sägepalme); Urtica dioica (Brennessel), insbesondere dessen Wurzel; Cucurbita pepo (Kürbis), insbesondere dessen Samen; Pygeum africanum; sowie deren geeignete Mischungen.

Eine bevorzugte Ausführungsform der erfindungsgemäßen pharmazeutischen Formulierung besteht darin, daß das Calciumsalz ausgewählt ist aus der Gruppe, bestehend aus:
Calciumcarbonaten; Calciumhydrogencarbonaten; Calciumhalogeniden, insbesondere Calciumchloriden und Calciumjodiden; Calciumphosphaten; Calciumhydrogenphosphaten, insbesondere Calciummonohydrogenphosphat; Dicalciumhydrogenphosphaten, Calciumlactaten; Calciumlactonaten; Calciumsuccinaten; Calciumtartraten; Calciumgluconaten; weitere; sowie deren geeignete Mischungen.

Bei der pharmazeutischen Formulierung gemäß der vorliegenden Erfindung kann zusätzlich noch Vitamin D₃ im Kern enthalten sein.

Auch ist es möglich, bei der vorliegenden pharmazeutischen Formulierung den Mantel aus mehreren Calciumschichten auszubilden, wobei vorzugsweise jede Schicht ein anderes Calciumsalz enthält.

Die pharmazeutische Formulierung weist einen Calciumgehalt von 50 bis 1000 mg pro Manteltablette und einen Pflanzentrockenextraktgehalt von 0,5 bis 100 mg auf. Hierdurch ist es möglich, daß mittels einer für den Patienten angenehmen ein- bis dreimaligen Gabe pro Tag die gesamte empfohlene Tagesdosis an Ca²⁺-lonen von etwa 1000 mg Ca²⁺ bei gleichzeitiger Gabe der erforderlichen Osteoporose-wirksamen Phytoextrakte sowie Vitamin D₃ zugeführt werden kann.

Ferner kann eine bevorzugte pharmazeutische Formulierung einen Polymerfilm als äußere Hülle aufweisen, weit hierdurch das Schluckverhalten und die Ösophagus-Gleitfähigkeit verbessert werden kann. Ferner kann der Polymerfilm Staubabrieb sowie das Eindringen von Feuchtigkeit in den Mantel verhindern. Jedoch ist eine derartige Polymerhülle in der Regel nicht erforderlich.

Die pharmazeutische Formulierung der vorliegenden Erfindung kommt mit wenig bis gar keinem Hilfsstoffen aus. Bei Bedarf können jedoch die in der Galenik üblichen Hilfsstoffe, beispielsweise Sprengmittel, insbesondere solche auf Basis von Stärken und/oder deren Derivaten; Bindemittel, insbesondere mikrokristalline Cellulose, Gummi arabicum; Schmiermittel, insbesondere Stearinsäure und/oder Magnesiumstearat; sowie Gleitmittel, insbesondere Polyethylenglykol, und dgl. verwendet werden.

Die erfindungsgemäßen pharmazeutischen Formulierungen eignen sich als Arzneimittel zur Behandlung von Osteoporose unterschiedlicher Genesen und besonders bevorzugt zur Prophylaxe und/oder Therapie von Sexualhormonmangel-bedingten Osteoporosen bei Frauen und Männern im fortgeschrittenen Alter.

Die Herstellung der erfindungsgemäßen pharmazeutischen Formulierungen erfolgt nach folgendem Prinzip:
Zunächst wird ein Kem-Preßling aus einem Pflanzentrockenextrakt hergestellt, wobei der Pflanzentrockenextrakt ausgewählt wird aus der Gruppe, bestehend aus Extrakten von: Vitex agnus castus (Mönchspfeffer); Belamcanda chinensis (Leopardenlilie); Cimicifuga racemosa (Traubensilberkerze); Trifolium pratense L. (Rotklee); Oenothera biennis hom. (Nachtkerze); Glycine soja (Sojabohne); Serenoa repens (Sägepalme); Urtica dioica (Brennessel), insbesondere dessen Wurzel; Cucurbita pepo (Kürbis), insbesondere dessen Samen; Pygeum africanum; sowie deren geeignete Mischungen.

Dieser Kern wird auf eine erste Teilmenge einer Mantelmischung aus wenigstens einem Calciumsalz transferiert, anschließend wird mit einer zweiten Teilmenge der Mantelmischung aufgefüllt; und die gesamte Formulierung aus Kern und den beiden Teilmengen der calciumsalzhaltigen Mantelmischung zu der pharmazeutischen Formulierung verpreßt. Das Calciumsalz wird ausgewählt aus der Gruppe, bestehend aus:
Calciumcarbonaten; Calciumhydrogencarbonaten; Calciumhalogeniden, insbesondere Calciumchloriden und Calciumjodiden; Calciumphosphaten; Calciumhydrogenphosphaten, insbesondere Calciummonohydrogenphosphat; Dicalciumhydrogenphosphaten, Calciumlactaten; Calciumlactonaten; Calciumsuccinaten; Calciumtartraten; Calciumgluconaten; weitere; sowie deren geeignete Mischungen.

Vorzugsweise wird der Kern auf der ersten Teilmenge der Mantelmischung im wesentlichen zentriert, wodurch eine sogenannte Manteltablette entsteht.

Zur Tablettenpressung werden handelsübliche Tablettenpressen beispielsweise solche der Firmen FETTE, KORSCH und KILIAN verwendet. Besonders geeignet ist beispielsweise eine synchron laufende Doppelrundlaufpresse der Firma MANESTY. Bei der Doppelrundlaufpresse werden die auf dem einen Turm der Rundlaufpresse hergestellt und durch den dazwischenliegenden Transfer- und Zentriermechanismus auf den Turm der Zweiten Rundlaufpresse überführt, wo das Aufpressen des Mantels erfolgt.

Grundsätzlich können die pharmazeutischen Formulierungen der vorliegenden Erfindung jedoch auch mit zwei Rundlaufpressen durchgeführt werden, wobei in einer die Kerne gepreßt werden, während in der zweiten der Mantel aufgepreßt wird.

Der Vorteil zur Herstellung der erfindungsgemäßen Manteltabletten als pharmazeutische Formulierung mittels einer Doppelrundlaufpresse liegt jedoch darin, daß die Kerne in dem ersten Turm der Presse relativ weich vorgepreßt werden können. Die Endkomprimierung im zweiten Turm der Doppelrundlaufpresse ergibt auf diese Weise eine sehr gute Haftung zwischen Pflanzentrockenextrakt-Kem und Calcium-Mantel.

Eine derart hergestellte Manteltablette weist beispielsweise folgende Zusammensetzung auf:

| | | |
|---|---|---|
| Mantel | CaCO₃ | 1250 mg (entsprechend 500mg Ca²⁺) |
| | Vitamin D₃ | 200 I.E. |
| Kern | Cimicifuga racemosa (Trockenextraktzubereitung) | 20 mg |
| Hilfsstoffe | Magnesiumstearat | 8 mg |
| | Stearinsäure | 4 mg |
| | mikrokristalline Celluose 40 mg | |
| | Gummi arabicum | 50 mg |

Die beispielhafte Manteltablette verbindet die Vorteile der oralen Calcium- und Vitamin D₃-Substitution mit der erforderlichen Gabe an Cimicifuga racemosa-Trockenextrakt. Durch orale Applikation von nur 2 Tabletten täglich wird die zur Osteoporoseprophylaxe und/oder - therapie empfohlene Calcium- und Vitamin D₃ - Dosis von ca. 1000 mg Ca²⁺ p.d. bzw. 400 I.E. Vitamin D₃ sowie die erwünschte gleichzeitige Gabe von 40 mg Cimicifuga racemosa Trockenextrakt erreicht.

Selbstverständlich ist es auch möglich und vorteilhaft je nach Indikationsstellung den Trockenextraktkem aus anderen der genannten Phytoextrakte oder auch aus Mischungen unterschiedlicher Extrakte herzustellen. So kann beispielsweise eine auf Prostataleiden abgestimmte erfindungsgemäße Manteltablette im Kern einen Serenoa repens-Extrakt enthalten

## Patentansprüche

1. Pharmazeutische Formulierung aus einem Calciumsalz und einem Pflanzentrockenextrakt,
**dadurch gekennzeichnet, daß**
daß die pharmazeutische Formulierung eine Manteltablette ist, wobei der Mantel ein Preßling aus wenigstens einem Calciumsalz und der Kern ein Preßling aus wenigstens einem Pflanzentrockenextrakt ist; und die Manteltablette einen Calciumgehalt von 50 - 1000 mg und einen Pflanzentrockenextraktgehalt von 0,5 - 100 mg aufweist.

2. Pharmazeutische Formulierung nach Anspruch 1, **dadurch gekennzeichnet, daß** der Pflanzentrockenextrakt ausgewählt ist aus der Gruppe, bestehend aus Extrakten von: Vitex agnus castus (Mönchspfeffer); Belamcanda chinensis (Leopardenlilie); Cimicifuga racemosa (Traubensilberkerze); Trifolium pratense L. (Rotklee); Oenothera biennis hom. (Nachtkerze); Glycine soja (Sojabohne); Serenoa repens (Sägepalme); Urtica dioica (Brennessel), insbesondere dessen Wurzel; Cucurbita pepo (Kürbis), insbesondere dessen Samen; Pygeum africanum; sowie deren geeignete Mischungen.

3. Pharmazeutische Formulierung nach Anspruch 1 oder 2, **dadurch gekennzeichnet, daß** das Calciumsalz ausgewählt ist aus der Gruppe, bestehend aus:
Calciumcarbonaten; Calciumhydrogencarbonaten; Calciumhalogeniden, insbesondere Calciumchloriden und Calciumjodiden; Calciumphosphaten; Calciumhydrogenphosphaten, insbesondere Calciummonohydrogenphosphat; Dicalciumhydrogenphosphaten, Calciumlactaten; Calciumlactonaten; Calciumsuccinaten; Calciumtartraten; Calciumgluconaten; sowie deren geeignete Mischungen.

4. Pharmazeutische Formulierung nach einem der Ansprüche 1 bis 3, **dadurch gekennzeichnet, daß** der Mantel mehrere Calciumschichten aufweist, wobei vorzugsweise jede Schicht ein anderes Calciumsalz enthält.

5. Pharmazeutische Formulierung nach einem der Ansprüche 1 bis 4, **dadurch gekennzeichnet, daß** sie zusätzlich einen Polymerfilm als äußere Hülle aufweist.

6. Pharmazeutische Formulierung nach einem der Ansprüche 1 bis 5, **dadurch gekennzeichnet, daß** sie einen Calciumgehalt von 1250 mg Calciumcarbonat (entsprechend ca. 500 mg Ca2+) und 200 I.E. Vitamin D3 im Mantel der Manteltablette sowie 20 mg Trockenextraktzubereitung aus Cimicifuga racemosa im Kern der Manteltablette aufweist.

7. Pharmazeutische Formulierung, nach einem der Ansprüche 1 bis 6, **dadurch gekennzeichnet, daß** sie im calciumhaltigen Mantel und/oder im inneren Pflanzentrockenextrakt-Preßling zusätzlich Cholecalciferole, insbesondere Cholecalciferol (Vitamin D3) enthält.

8. Pharmazeutische Formulierung nach einem der Ansprüche 1 bis 7, **dadurch gekennzeichnet, daß** sie zusätzlich wenigstens ein Fluoridsalz, insbesondere Natriumfluorid enthält.

9. Pharmazeutische Formulierung nach Anspruch 8, **dadurch gekennzeichnet, daß** das Fluoridsalz im inneren Preßling vorliegt.

10. Pharmazeutische Formulierung nach einem der Ansprüche 1 bis 9, **dadurch gekennzeichnet, daß** sie als Hilfsstoffe Sprengmittel, insbesondere solche auf Basis von Stärken und/oder deren Derivaten; Bindemittel, insbesondere mikrokristalline Cellulose, Gummi arabicum; Schmiermittel, insbesondere Stearinsäure und/oder Magnesiumstearat; sowie Gleitmittel, insbesondere Polyethylenglykol, aufweist.

11. Verwendung von einem calciumsalz und einem Pflanzentrockenextrakt zur Herstellung einer pharmazeutischen Formulierung gemäß einem der Ansprüche 1 bis 10 zur Behandlung von Osteoporose unterschiedlicher Genese.

12. Verwendung nach Anspruch 11, **dadurch gekennzeichnet, daß** die pharmazeutische Formulierung als Arzneimittel zur Behandlung von Sexualhormonmangelbedingten Osteoporosen zur Prävention eingesetzt wird.

13. Verfahren zur Herstellung einer pharmazeutischen Formulierung gemäß einem der Ansprüche 1 bis 10, **dadurch gekennzeichnet, daß**
man einen Kern-Preßling aus einem Pflanzentrockenextrakt herstellt, wobei der Pflanzentrockenextrakt ausgewählt wird aus der Gruppe, bestehend aus Extrakten von: Vitex agnus castus (Mönchspfeffer); Belamcanda chinensis (Leopardenlilie); Cimicifuga racemosa (Traubensilberkerze); Trifolium pratense L. (Rotklee); Oenothera biennis hom. (Nachtkerze); Glycine soja (Sojabohne); Serenoa repens (Sägepalme); Urtica dioica (Brennessel), insbesondere dessen Wurzel; Cucurbita pepo (Kürbis), insbesondere dessen Samen; Pygeum africanum; sowie deren geeignete Mischungen;
man den Kern auf eine erste Teilmenge einer Mantelmischung aus wenigstens einem Calciumsalz transferiert, anschließend mit einer zweiten Teilmenge der Mantelmischung auffüllt; und
man die gesamte Formulierung aus Kern und den beiden Teilmengen der calciumsalzhaltigen Mantelmischung zu der pharmazeutischen Formulierung verpreßt;
wobei das Calciumsalz ausgewählt wird aus der Gruppe, bestehend aus:
Calciumcarbonaten; Calciumhydrogencarbonaten; Calciumhalogeniden, insbesondere Calciumchloriden und Calciumjodiden; Calciumphosphaten; Calciumhydrogenphosphaten, insbesondere Calciummonohydrogenphosphat; Dicalciumhydrogenphosphaten, Calciumlactaten; Calciumlactonaten; Calciumsuccinaten; Calciumtartraten; Calciumgluconaten; sowie deren geeignete Mischungen.

14. Verfahren nach Anspruch 13, **dadurch gekennzeichnet, daß** man den Kern auf der ersten Teilmenge der Mantelmischung zentriert.

15. Verfahren nach Anspruch 13 oder 14, **dadurch gekennzeichnet, daß** man als Hilfsstoffe Sprengmittel, insbesondere solche auf Basis von Stärken und/oder deren Derivaten; Bindemittel, insbesondere mikrokristalline Cellulose, Gummi arabicum; Schmiermittel, insbesondere Stearinsäure und/oder Magnesiumstearat; sowie Gleitmittel, insbesondere Polyethylenglykol, verwendet.

## Claims

1. Pharmaceutical formulation of a calcium salt and a dry plant extract, **characterized in that** the pharmaceutical formulation is a coated tablet, wherein the coating is a pressed body of at least one calcium salt, and the core is a pressed body of at least one dry plant extract and the coated tablet has a calcium content of 50 to 1000 mg and a dry plant extract content of 0.5 to 100 mg.

2. Pharmaceutical formulation in accordance with claim 1, **characterized in that** the dry plant extract is selected from the group consisting of extracts from:
*Vitex agnus castus* (chaste tree), *Belamcanda chinensis* (leopard lily), *Cimicifuga racemosa* (black cohosh), *Trifolium pratense L.* (purple trefail), *Oenothera biennis hom.* (primrose), *Glycine soja* (soy bean), *Serenoa repens* (saw-palmetto), *Urtica dioica* (stinging nettle), in particular its root, *Cucurbita* pepo (pumpkin), in particular its seed, *Pygeum africanum,* as well as suitable mixtures of these.

3. Pharmaceutical formulation in accordance with claim 1 or 2, **characterized in that** the calcium salt is selected from the group consisting of: calcium carbonates, calcium hydrogen carbonates, calcium halides, in particular calcium chlorides and calcium iodides, calcium phosphates, calcium hydrogen phosphates, in particular calcium monohydrogen phosphate, dicalcium hydrogen phosphates, calcium lactates, calcium lactonates, calcium succinates, calcium tartrates, calcium gluconates, as well as suitable mixtures of these.

4. Pharmaceutical formulation in accordance with any one of claims 1 to 3, **characterized in that** the coating includes a plurality of calcium layers, wherein preferably each layer contains a different calcium salt,

5. Pharmaceutical formulation in accordance with any one of claims 1 to 4, **characterized in that** it additionally has a polymer film as an external envelope.

6. Pharmaceutical formulation in accordance with any one of claims 1 to 5, **characterized in that** it has a calcium content of 1250 mg of calcium Carbonate (corresponding to approx. 500 mg of Ca2+) and 200 I. U. vitamin D₃, in the coating of the coated tablet as well as 20 mg dry extract preparation of *Cimicifuga racemosa* in the core of the coated tablet.

7. Pharmaceutical formulation in accordance with any one of claims 1 to 6, **characterized in that** in the calcium-containing coating and/or in the inner pressed body of dry plant extract it additionally contains cholecalciferoles, in particular Colecalciferol (vitamin D₃).

8. Pharmaceutical formulation in accordance with any one of claims 1 to 7, **characterized in that** it additionally contains at least one fluoride salt, in particular sodium fluoride.

9. Pharmaceutical formulation in accordance with claim 8, **characterized in that** the fluoride salt is present in the inner pressed body.

10. Pharmaceutical formulation in accordance with any one of claims 1 to 9, **characterized in that** by way of auxiliaries it includes disintegrants, in particular on the basis of starches and/or their derivatives, binders, in particular microcrystalline cellulose, gum arabic, lubricants, in particular stearic acid and/or magnesium stearate, as well as slip agents, in particular polyethylene glycol.

11. Use of a calcium salt and a dry plant extract for manufacturing a pharmaceutical formulation in accordance with any one of claims 1 to 10 for the treatment of osteoporoses of various geneses.

12. Use in accordance with Claim 11, **characterized in that** the pharmaceutical formulation is used as a medicament for the treatment of osteoporoses brought about by lack of sexual hormone as a prevention.

13. Method for manufacturing a pharmaceutical formulation in accordance with any one of claims 1 to 10, **characterized in that** a pressed body core is manufactured of a dry plant extract, wherein the dry plant extract is selected from the group consisting of extracts from: *Vitex agnus castus* (chaste tree), *Belamcanda chinensis* (leopard lily), *Cimicifuga racemosa* (black cohosh), *Trifolium pratense L.* (purple trefail), *Oenothera biennis hom.* (primrose), *Glycine soja* (soy bean), *Serenoa repens* (saw-palmetto), *Urtica dioica* (stinging nettle), in particular its root, *Cucurbita pepo* (pumpkin), in particular its seed, *Pygeum africanum,* as well as suitable mixtures of these, the core is transferred to a first partial quantity of a coating mixture of at least one calcium salt, which is followed by filling with a second partial quantity of the coating mixture, and the entire formulation comprised of the core and the two partial quantities of the calcium salt-containing coating mixture is pressed to form the pharmaceutical formulation, wherein the calcium salt is selected from the group consisting of:
calcium Carbonates, calcium hydrogen Carbonates, calcium halides, in particular calcium chlorides and calcium chlorides, calcium phosphates, calcium hydrogen phosphates, in particular calcium monohydrogen phosphate, dicalcium hydrogen phosphates, calcium lactates, calcium lactonates, calcium succinates, calcium tartrates, calcium gluconates, as well as suitable mixtures of these.

14. Method in accordance with claim 13, **characterized in that** the core is centered on the first partial quantity of the coating mixture.

15. Method in accordance with claim 13 or 14, **characterized in that** auxiliaries used are disintegrants, in particular on the basis of starches and/or their derivatives, binders, in particular microcrystalline cellulose, gum arabic, lubricants, in particular stearic acid and/or magnesium stearate, as well as slip agents, in particular polyethylene glycol.

## Revendications

1. Formulation pharmaceutique à base de sel de calcium et d'un extrait sec de plante, **caractérisé en ce que** la formulation pharmaceutique est un comprimé enrobé, où le revêtement étant un produit pressé constitué d'au moins un sel de calcium et le noyau étant un produit pressé constitué d'au moins un extrait sec de plante et le comprimé enrobé présente une teneur en calcium de 50 à 1000 mg et une teneur en extrait sec de plante de 0,5 à 100 mg.

2. Formulation pharmaceutique selon la revendication 1, **caractérisée en ce que** l'extrait sec de plante est choisi dans le groupe composé des extraits de, *Vitex agnus castus* (arbre au poivre), *Belamcanda chinensis* (iris tigré), *Cimicifuga racemosa* (actée à grappes), *Trifolium pratense L.* (trèfle rouge), *Oenothera biennis hom.* (onagre), *Glycine soja* (grai nes de soja), *Serenoa repens* (palmiste), *Urtica dioica* (ortie), en particulier ses racines, *Cucurbita pepo* (courge), en particulier ses graines, *Pygeum africanum,* ainsi que leurs mélanges appropriés.

3. Formulation pharmaceutique selon la revendication 1 ou 2, **caractérisée en ce que** le sel de calcium est choisi dans le groupe composé de :
es carbonates de calcium, les hydrogénocarbonates de calcium, les halogénures de calcium, en particulier les chlorures de calcium et les iodures de calcium, les phosphates de calcium, les hydrogénophosphates de calcium, en particulier les rnonohydrogénophosphates de calcium, les hydrogénophosphates dicalciques, les lactates de calcium, les lactonates de calcium, les succinates de calcium, les tartrates de calcium, les gluconates de calcium, ainsi que leurs mélanges appropriés.

4. Formulation pharmaceutique selon l'une quelconque des revendications 1 à 3, **caractérisée en ce que** le revêtement presente plusieurs couches de calcium, de preference, chaque couche contenant un autre sel de calcium.

5. Formulation pharmaceutique selon l'une quelconque des revendications 1 à 4, **caractérisée en ce qu'**elle présente en outre un film polymère comme enveloppe externe.

6. Formulation pharmaceutique selon l'une quelconque des revendications 1 à 5, **caractérisée en ce qu'**elle présente une teneur en calcium de 1250 mg de carbonate de calcium (correspondant environ à 500 mg Ca²⁺) et 200 Ul de vitamine D3 dans le revêtement du comprimé enrobé ainsi que 20 mg de préparation d'extrait sec de Cimicifuga racemosa dans le noyau du comprimé enrobé.

7. Formulation pharmaceutique selon l'une quelconque des revendications 1 à 6, **caractérisée en ce qu'**elle contient en outre dans le revêtement contenant du calcium et/ou dans le produit pressé interne d'extrait sec de plante, des cholécalciférols, en particulier du colécalciférol (vitamine D3).

8. Formulation pharmaceutique selon l'une quelconque des revendications 1 à 7, **caractérisée en ce qu'**elle contient en outre, au moins un sel de fluorure, en particulier du fluorure de sodium.

9. Formulation pharmaceutique selon la revendication 8, **caractérisée en ce que** le sel de fluorure est présent dans le produit pressé interne.

10. Formulation pharmaceutique selon l'une quelconque des revendications 1 à 9, **caractérisée en ce qu'**elle présente, comme adjuvant, des dispersants, en particulier ceux à base d'amidon et/ou leurs dérivés : des agents liants, en particulier de la cellulose microcristalline, de la gomme arabique, des lubrifiants, en particulier de l'acide stéarique et/ou du stéarate de magnésium, ainsi que des agents glissants, en particulier du polyéthyléne-glycol.

11. Utilisation d'un sel de calcium et d'un extrait sec de plante pour la fabrication d'une formulation pharmaceutique selon l'une quelconque des revendications 1 à 10 pour le traitement de l'ostéoporose d'origines diverses.

12. Utilisation selon la revendication 11, **caractérisée en ce que** la formulation pharmaceutique est utilisée comme médicament pour le traitement des ostéoporoses dues aux hormones sexuelles, en prévention.

13. Procéde de fabrication d'une formulation pharmaceutique selon l'une quelconque des revendications 1 à 10, **caractérisée ce que** l'on produit un produit pressé noyau à base d'un extrait sec de plante, l'extrait sec de plante étant choisi dans le groupe constitué des extraits de , *Vitex agnus castus* (arbre au poivre), *Belamcanda chinensis* (iris tigré), *Cimicifuga racemosa* (actée à grappes), *Trifolium pratense L.* (trèfle rouge), *Oenothera biennis hom.* (onagre), *Glycine soja* (graines de soja), *Serenoa repens* (palmiste), *Urtica dioica* (ortie), en particulier ses racines, *Cucurbita pepo* (courge), en particulier ses graines, *Pygeum africanum,* ainsi que leurs mélanges appropriés, on transfère le noyau sur une première quantité partielle d'un mélange de revêtement comprenant au moins un sel de calcium, ensuite on complète avec une deuxième quantité partielle du mélange de revêtement, et on compresse la formulation complète constituée du noyau et des deux quantités partielles de mélange de revêtement contenant du sel de calcium en ladite formulation pharmaceutique, le sel de calcium étant choisi dans le groupe composé de :
les carbonates de calcium, les hydrogéncarbonates de calcium, les halogénures de calcium, en particulier les chlorures de calcium et les iodures de calcium, les phosphates de calcium, les hydrogénophosphates de calcium, en particulier les rnonohydrogénocarbonate de calcium, les hydrogénophosphates dicalciques, les lactates de calcium, les lactonates de calcium, les succinates de calcium, les tartrates de calcium, les gluconates de calcium, ainsi que leurs mélanges appropriés.

14. Procédé selon la revendication 13, **caractérisée en ce que** l'on centre le noyau sur la première quantité partielle de mélange de revêtement.

15. Procédé selon la revendication 13 ou 14, **caractérisée en ce que** l'on utilise comme adjuvants, des dispersants, en particulier ceux à base d'amidon et/ou leurs dérivés, des agents liants, en particulier de la cellulose microcristalline, de la gomme arabique, des lubrifiants, en particulier de l'acide stéarique et/ou du stéarate de magnésium, ainsi que des agents glissants, en particulier du polyéthylène-glycol.
